# FASCICULE DE BREVET EUROPEEN

(11) **EP 2 086 565 B1**
(45) Date de publication et mention de la délivrance du brevet: **07.05.2014**
(21) Numéro de dépôt: 07866450.5
(22) Date de dépôt: 26.10.2007
(51) Int. Cl.: A61K 38/08, A61K 38/07, A61K 8/64, A61Q 17/04, A61Q 19/04, A61P 17/00

(54) **COMPOSITION COSMETIQUE ET/OU PHARMACEUTIQUE COMPRENANT COMME PRINCIPE ACTIF AU MOINS UN PEPTIDE ET UTILISATION DE CE PEPTIDE**
KOSMETISCHE UND/ODER PHARMAZEUTISCHE ZUSAMMENSETZUNG MIT MINDESTENS EINEM PEPTID ALS WIRKSTOFF UND VERWENDUNG DES PEPTIDS
COSMETIC AND/OR PHARMACEUTICAL COMPOSITION CONTAINING AT LEAST ONE PEPTIDE AS THE ACTIVE INGREDIENT AND USE OF SAID PEPTIDE

(30) Priorité: 26.10.2006 FR 0609416
(43) Date de publication de la demande: 12.08.2009
(73) Titulaire: Ashland Specialties France, 06410 Biot (FR)
(72) Inventeur: DAL FARRA, Claude, 06650 Opio (FR); DOMLOGE, Nouha, 06560 Valbonne (FR); BOTTO, Jean-Marie, 06560 Valbonne (FR)
(74) Mandataire: Macquet, Christophe
(86) Numéro de dépôt international: PCT/FR2007/001777
(87) Numéro de publication internationale: WO 2008/062110

(56) Documents cités:
- WO-A-00/01841
- US-B1- 6 268 342

### SEQUENCE LISTING

<110> Société d'Extraction des principes Actifs
<120> COMPOSITION COSMETIQUE ET/OU PHARMACEUTIQUE COMPRENANT COMME PRINCIPE ACTIF AU MOINS UN PEPTIDE ET UTILISATION DE CE PEPTIDE
<130> Bv 06-79
<150> FR 06 09416
   <151> 2006-10-26
<160> 8
<170> PatentIn version 3.3
<210> 1
   <211> 8
   <212> PRT
   <213> Artificial
<220>
   <223> synthetic peptide
<400> 1
   Asn Gly Trp Lys Ile Glu Arg Lys
   1               5
<210> 2
   <211> 6
   <212> PRT
   <213> Artificial
<220>
   <223> synthetic peptide
<400> 2
   Asn Gly Trp Arg Val Asp
   1               5
<210> 3
   <211> 6
   <212> PRT
   <213> Artificial
<220>
   <223> synthetic peptide
<400> 3
   Trp Arg Leu Asp Arg Lys
   1               5
<210> 4
   <211> 4
   <212> PRT
   <213> Artificial
<220>
   <223> synthetic peptide
<400> 4
   Trp Lys Leu Asp
   1
<210> 5
   <211> 4
   <212> PRT
   <213> Artificial
<220>
   <223> synthetic peptide
<400> 5
   Trp Arg Val Glu
   1
<210> 6
   <211> 4
   <212> PRT
   <213> Artificial
<220>
   <223> synthetic peptide
<400> 6
   Trp His Leu Glu
   1
<210> 7
   <211> 4
   <212> PRT
   <213> Artificial
<220>
   <223> synthetic peptide
<400> 7
   Trp Arg Ala Asp
   1
<210> 8
   <211> 4
   <212> PRT
   <213> Artificial
<220>
   <223> synthetic peptide
<400> 8
   Trp Lys Ile Asp
   1

## Description

La présente invention se situe dans le domaine cosmétique et pharmaceutique, et plus particulièrement dans le domaine de la dermatologie. La présente invention concerne une composition cosmétique et/ou pharmaceutique et/ou dermatologique contenant, comme principe actif, au moins un peptide de séquence SEQ ID n°1 à SEQ ID n°8.

Le principe actif selon l'invention est destiné à induire, augmenter ou restaurer la synthèse de mélanine dans les mélanocytes de l'épiderme ou du bulbe pileux, en vue de donner à la peau un aspect bronzé, de la préparer à une exposition au soleil, ou de la protéger des rayonnements UV (ultra-violets). Le principe actif peut également être utilisé à des fins thérapeutiques, pour préparer des compositions pharmaceutiques destinées à repigmenter la peau dépigmentée par exemple dans le cas du vitiligo, ou pour pigmenter les poils ou les cheveux, en particulier dans le traitement et la prévention de la canitie.

Chez l'humain, la couleur des cheveux et de la peau est liée à des facteurs individuels (origine ethnique, sexe, âge, etc.) et à des facteurs environnementaux (notamment les saisons de l'année, la zone d'habitation, etc.). Elle est principalement déterminée par la nature et la concentration de mélanine produite par les mélanocytes. La mélanine a la propriété de protéger les cellules cutanées des effets délétères des rayonnements UV et de ralentir le photo-vieillissement cutané. Les mélanocytes sont les cellules spécialisées qui, par l'intermédiaire d'organites particuliers, les mélanosomes, synthétisent la mélanine. La synthèse de la mélanine ou mélanogénèse est un processus complexe dont les mécanismes précis ne sont pas encore élucidés et qui fait intervenir schématiquement les étapes suivantes :

Tyrosine → Dopa → Dopaquinone → Dopachrome → Mélanine

Dans l'épiderme, le mélanocyte est impliqué dans l'unité mélanique épidermique qui comporte un mélanocyte entouré d'environ 40 kératinocytes voisins. Au fur et à mesure que la mélanine est synthétisée dans les mélanosomes, ceux-ci se déplacent de la région périnucléaire vers l'extrémité des dendrites des mélanocytes. Par phagocytose, l'extrémité des dendrites est capturée par les kératinocytes, les membranes dégradées et les mélanosomes redistribués dans les kératinocytes, où ils assureront une protection jusqu'à la desquamation naturelle des cellules. La production de mélanine, ainsi que son transport, sont régulés par différents facteurs tels que, par exemple, les rayonnements UV, les hormones ou les produits chimiques. Ainsi, une augmentation de l'exposition aux rayonnements UV provoque la synthèse de pigments et le brunissement de la peau, qui a pour effet de protéger la peau des rayonnements UV.

La pigmentation naturelle des cheveux et des poils par la mélanine requiert la présence de mélanocytes au niveau du bulbe du follicule pileux. Le follicule pileux est une invagination tubulaire de l'épiderme qui s'enfonce jusqu'aux couches profondes du derme. Les mélanocytes au niveau du bulbe du follicule pileux sont dans un état actif, c'est-à-dire qu'ils synthétisent de la mélanine. Ces pigments sont transmis aux kératinocytes destinés à former la tige pilaire, ce qui conduira à la pousse d'un cheveu ou d'un poil pigmenté.

Il est admis que la canitie (blanchiment naturel des cheveux) est associée à une diminution de mélanine dans la tige pilaire.

La recherche de composés pouvant favoriser la synthèse de la mélanine dans la peau et les cheveux, en l'absence de stimulation par les UV, est une préoccupation de la dermatologie et de la cosmétique. Ces nouveaux composés seraient particulièrement utiles comme alternative aux expositions solaires, pour préparer la peau et protéger celle-ci des rayons du soleil, pour obtenir un bronzage plus intense après une exposition au soleil, pour prolonger la pigmentation naturelle de la peau après une exposition au soleil ou pour prévenir et/ou limiter et/ou stopper le développement de la canitie et même maintenir la pigmentation naturelle des cheveux et/ou des poils gris ou blancs.

On entend par pigmentation naturelle de la peau, la coloration de la peau ou des cheveux déterminée par la concentration en mélanine.

Par poils et cheveux on entend l'ensemble des annexes pileuses et notamment également les cils et les sourcils.

A cet égard, il a été proposé de nombreuses solutions par apport de colorants exogènes, dont le plus connu est la dihydroxyacétone, ou DHA. Toutefois, seule la stimulation de la pigmentation de la peau et/ou des cheveux par la voie naturelle permet une réelle protection vis à vis des rayonnements UV et reste la voie idéale de stimulation de la pigmentation. Ainsi il a été proposé dans l'art antérieur la préparation et l'utilisation d'activateurs de la biosynthèse de la mélanine (FR828097, FR 2831438, FR 2845285) faisant intervenir des hormones (alpha MSH ou ses dérivés WO2006037188) ou des prostaglandines (WO9511003).

D'autre part, l'utilisation de composés favorisant la synthèse de la mélanine dans la peau et les cheveux est tout particulièrement intéressante pour traiter les pathologies provoquant des hypopigmentations localisées, d'origines génétiques, auto-immunes comme le vitiligo, dues au vieillissement, ou encore post-lésionnelles (cicatrices, mycoses).

La présente invention a pour principal objectif de fournir un nouveau principe actif capable d'induire, d'augmenter ou de restaurer la synthèse de mélanine dans les mélanocytes de l'épiderme et du bulbe pileux. Les inventeurs ont en effet mis en évidence que les peptides de séquences SEQ ID n°1 à n°8 ont des propriétés remarquables, et notamment que ces peptides, lorsqu'ils sont appliqués sur la peau, favorisent de façon importante la synthèse de mélanine dans les mélanocytes de l'épiderme ou du bulbe pileux. Ces nouveaux principes actifs permettent ainsi d'ouvrir de nouvelles perspectives thérapeutiques et cosmétiques.

Ainsi, l'invention a pour objet premier l'utilisation de peptides de séquence :
(SEQ ID n°1) Asn-Gly-Trp-Lys-Ile-Glu-arg-Lys
(SEQ ID n°2) Asn-Gly-Trp-Arg-Val-Asp
(SEQ ID n°3) Trp-Arg-Leu-Asp-Arg-Lys
(SEQ ID n°4) Trp-Lys-Leu-Asp
(SEQ ID n°5) Trp-Arg-Val-Glu
(SEQ ID n°6) Trp-His-Leu-Glu
(SEQ ID n°7) Trp-Arg-Ala-Asp
(SEQ ID n°8) Trp-Lys-Ile-Asp

Selon un mode de réalisation particulièrement intéressant, le peptide actif correspond à la séquence SEQ ID n°4.

Selon un autre mode de réalisation particulièrement intéressant, le peptide actif correspond à la séquence SEQ ID n°5.

Dans l'invention, le terme "acide aminé" se réfère ici à tout acide organique naturel ou non naturel ayant la formule :

NHR-CR-C(O)-O-

où chaque -R est indépendamment sélectionné entre un hydrogène et un groupement alkyl ayant entre 1 et 12 atomes de carbone. Préférentiellement, au moins un groupement -R de chaque acide aminé est un hydrogène. Par le terme "alkyl", on entend ici une chaîne carbonée pouvant être linéaire ou ramifiée, substituée (mono- ou poly-) ou non-substituée ; saturée, mono-saturée (une double ou triple liaison dans la chaîne) ou poly-insaturée (deux ou plusieurs doubles liaisons, deux ou plusieurs triples liaisons, une ou plusieurs doubles liaisons et une ou plusieurs triples liaisons dans la chaîne).

Le terme « peptide » désigne un enchaînement de deux ou plusieurs acides aminés liés entre eux par des liaisons peptidiques ou par des liaisons peptidiques modifiées. Par « peptide », il faut entendre le peptide naturel ou synthétique de l'invention tel que décrit ci-dessus ou au moins l'un de ses fragments, qu'il soit obtenu par protéolyse ou de manière synthétique ou encore tout peptide naturel ou synthétique dont la séquence est totalement ou partiellement constituée par la séquence du peptide précédemment décrit.

De façon à améliorer la résistance à la dégradation, il peut être nécessaire d'utiliser une forme protégée du peptide selon l'invention. La forme de protection doit évidemment être une forme biologiquement compatible et doit être compatible avec une utilisation dans le domaine des cosmétiques ou de la pharmacie.

De nombreuses formes de protection biologiquement compatibles peuvent être envisagées, elles sont bien connues de l'homme du métier comme, par exemple, l'acylation ou l'acétylation de l'extrémité amino-terminale, ou l'amidation ou l'estérification de l'extrémité carboxy-terminale. Ainsi, l'invention concerne une utilisation telle que définie précédemment, caractérisée par le fait que le peptide est sous forme protégée ou non. De préférence, on utilise une protection basée soit sur l'acylation ou l'acétylation de l'extrémité amino-terminale, soit sur l'amidation ou l'estérification de l'extrémité carboxy-terminale, soit encore des deux. Les dérivés d'acides aminés et les dérivés de peptides concernent aussi les acides aminés et les peptides reliés entre eux par une liaison pseudo-peptidique. On entend par "liaison pseudo-peptidique" tous les types de liaisons susceptibles de remplacer les liaisons peptidiques "classiques".

Dans le domaine des acides aminés, la géométrie des molécules est telle qu'elles peuvent théoriquement se présenter sous la forme d'isomères optiques différents. Il existe, en effet, une conformation moléculaire de l'acide aminé (AA) telle qu'elle dévie à droite le plan de polarisation de la lumière (conformation dextrogyre ou D-aa), et une conformation moléculaire de l'acide aminé (aa) telle qu'elle dévie à gauche le plan de polarisation de la lumière (conformation lévogyre ou L-aa). La nature n'a retenu pour les acides aminés naturels que la conformation lévogyre. En conséquence, un peptide d'origine naturelle ne sera constitué que d'acides aminés de type L-aa. Cependant la synthèse chimique en laboratoire permet de préparer des acides aminés ayant les deux conformations possibles. A partir de ce matériel de base, il est ainsi possible d'incorporer lors de la synthèse de peptide aussi bien des acides aminés sous forme d'isomères optiques dextrogyre ou lévogyre. Ainsi, les acides aminés constituant le peptide selon l'invention peuvent être sous configuration L- et D- ; de manière préférentielle, les acides aminés sont sous forme L. Le peptide selon l'invention peut donc être sous forme L-, D- ou DL-.

Le peptide, objet du présent brevet, peut être obtenu soit par synthèse chimique classique (en phase solide ou en phase homogène liquide), soit par synthèse enzymatique *(*Kullman et al., J Biol. Chem. 1980, 225, 8234*)* à partir d'acides aminés constitutifs ou de leurs dérivés.

Le peptide selon l'invention peut également être obtenu par fermentation d'une souche de bactéries modifiées ou non, par génie génétique pour produire les peptides de séquences SEQ ID n°1 à n°8, ou encore par extraction de protéines d'origine animale ou végétale, préférentiellement d'origine végétale, suivie d'une hydrolyse contrôlée qui libère les fragments peptidiques de tailles moyennes et de petites tailles, objet de l'invention.

De très nombreuses protéines trouvées dans les plantes sont susceptibles de contenir ces séquences au sein de leur structure. L'hydrolyse ménagée permet de dégager ces fragments peptidiques. Il est possible, mais non nécessaire pour réaliser l'invention, d'extraire soit les protéines concernées d'abord et de les hydrolyser ensuite, soit d'effectuer l'hydrolyse d'abord sur un extrait brut et de purifier les fragments peptidiques ensuite. Il est également possible d'utiliser certains extraits hydrolysés sans en purifier les fragments peptidiques selon l'invention, mais en s'assurant toutefois de la présence desdits fragments par des moyens analytiques appropriés.

D'autres procédés plus simples ou plus complexes peuvent être envisagés par l'homme du métier connaissant le métier de synthèse, d'extraction et de purification des protéines et des peptides. Ainsi le peptide selon l'invention peut être d'origine naturelle ou synthétique. Préférentiellement selon l'invention, le peptide est obtenu par synthèse chimique.

Dans la composition selon l'invention, les peptides peuvent être un mélange de dérivés peptidiques et/ou constitués de dérivés d'acides aminés.

Selon un mode de réalisation avantageux de l'invention, les peptides de séquences SEQ ID n°1 à n°8 sont préalablement solubilisés dans un ou plusieurs solvants cosmétiquement ou pharmaceutiquement acceptables, classiquement utilisés par l'homme du métier, comme l'eau, le glycérol, l'éthanol, le propylène glycol, le butylène glycol, le dipropylène glycol, les diglycols éthoxylés ou propoxylés, les polyols cycliques, la vaseline, une huile végétale ou tout mélange de ces solvants.

Selon encore un autre mode de réalisation avantageux de l'invention, les peptides de séquences SEQ ID n°1 à n°8 sont préalablement solubilisés dans un vecteur cosmétique ou pharmaceutique comme les liposomes ou adsorbés sur des polymères organiques poudreux, des supports minéraux comme les talcs et bentonites, et plus généralement solubilisés dans, ou fixés sur, tout vecteur cosmétiquement ou pharmaceutiquement acceptable.

Dans la composition selon l'invention, le peptide peut être un mélange de dérivés peptidiques et/ou constitué de dérivés d'acides aminés. Il est bien entendu que le peptide selon l'invention peut être utilisé seul ou en association avec au moins un autre principe actif.

Les compositions selon l'invention pourront être appliquées par toute voie appropriée, notamment orale, parentérale ou topique externe, et leur formulation sera adaptée par l'homme du métier, en particulier pour des compositions cosmétiques ou dermatologiques. Avantageusement, les compositions selon l'invention sont destinées à une administration par voie topique cutanée. Elles contiennent un milieu physiologiquement acceptable, en particulier un milieu cosmétologiquement ou pharmaceutiquement, notamment dermatologiquement acceptable, et couvrent toutes les formes cosmétiques ou dermatologiques. Ces compositions doivent donc contenir un milieu cosmétiquement et/ou dermatologiquement acceptable, c'est-à-dire compatible avec la peau, les poils ou les cheveux. Ces compositions pourront notamment être sous forme de crèmes, émulsions huile-dans-eau, ou eau-dans-huile ou émulsions multiples, solutions, suspensions, gels, laits, lotions, sticks ou encore poudres, adaptés à une application sur la peau, les lèvres et/ou les cheveux.

Ces compositions comprennent les excipients nécessaires à leur formulation, tels que solvants, épaississants, diluants, tensioactifs, anti-oxydants, colorants, conservateurs, parfums.

Bien entendu, l'homme de métier veillera à choisir les éventuels composés complémentaires, actifs ou non-actifs, et/ou leur quantité, de telle sorte que les propriétés avantageuses du mélange ne soient pas, ou sensiblement pas, altérées par l'adjonction envisagée.

La composition utilisable selon l'invention peut en particulier consister en une composition pour soins capillaires, et notamment un shampooing, un après-shampooing, une lotion de mise en plis, une lotion traitante, une crème ou un gel coiffant, une lotion restructurante pour les cheveux, un masque, etc. La composition cosmétique selon l'invention peut être utilisée notamment dans les traitements mettant en oeuvre une application qui est suivie ou non suivie d'un rinçage, ou encore sous forme de shampooing.

Elle peut également se présenter sous forme de teinture ou de mascara à appliquer au pinceau ou au peigne, en particulier sur les cils, les sourcils ou les cheveux.

Il est bien entendu que le peptide selon l'invention peut être utilisé, en tant que principe actif, seul ou bien en association avec au moins un autre principe actif, dans une composition cosmétique ou pour la préparation d'une composition dermatologique et/ou pharmaceutique. Avantageusement, les compositions utilisables selon l'invention contiennent en outre au moins un autre composé favorisant la pigmentation de la peau, des cheveux et/ou des poils.

De tels composés sont notamment des substrats de la tyrosinase, tels que la tyrosine ou la L-DOPA, des prostaglandines ou des composés activateurs de la voie de l'AMPc tels que des dérivés de pro-opiomélanocortines, l'adénosine, ou la forskoline ou ses dérivés. On peut également citer des extraits de végétaux tels que le bigaradier (*citrus aurantium*) ou de chrysanthème (*Chrysanthemum morifolium*), décrits notamment dans les brevets FR 2845285 et EP 1014934.

De tels composés se trouvent également dans la famille des colorants exogènes des couches superficielles de l'épiderme, tels que la dihydroxyacétone (DHA), l'érythrulose, les extraits de feuilles de henné, décrits notamment dans les brevets EP 0742002 et FR2779958.

Il est bien évident que l'invention s'adresse aux mammifères en général et plus particulièrement aux êtres humains.

La quantité efficace de principe actif correspond à la quantité nécessaire afin d'obtenir le résultat désiré. Selon un mode de réalisation avantageux de l'invention, le peptide précité est présent dans les compositions de l'invention à une concentration comprise entre 0,0005 et 500 ppm (parties par million) environ, et préférentiellement à une concentration comprise entre 0,01 et 5 ppm environ par rapport au poids total de la composition finale.

Ces compositions pourront notamment se présenter sous forme d'une solution aqueuse, hydroalcoolique ou huileuse ; d'une émulsion huile-dans-eau, eau-dans-huile ou émulsions multiples ; elles peuvent aussi se présenter sous forme de crèmes, de suspensions, ou encore de poudres, adaptées à une application sur la peau, les muqueuses, les lèvres et/ou les phanères. Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent aussi se présenter sous forme solide, comme un stick, ou être appliquées sur la peau sous forme d'aérosol. Elles peuvent être utilisées comme produit de soin et/ou comme produit de maquillage de la peau.

Ces compositions comprennent, en outre, tout additif communément utilisé dans le domaine d'application envisagé ainsi que les adjuvants nécessaires à leur formulation, tels que des solvants, des épaississants, des diluants, des anti-oxydants, des colorants, des filtres solaires, des composés auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc.

Dans tous les cas, l'homme du métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre de 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Un autre objet de l'invention consiste en une composition cosmétique ou dermatologique, caractérisée en ce qu'elle contient, dans un milieu cosmétiquement ou dermatologiquement acceptable, le composé afin de préparer la peau à une exposition au soleil et de la protéger des rayonnements du soleil.

L'invention consiste encore en une composition pharmaceutique caractérisée en ce que le composé est formulé pour atténuer une pathologie liée à la pigmentation telle que le vitiligo qui se traduit par une hypopigmentation localisée de la peau.

L'invention a également pour objet l'utilisation dans une composition cosmétique, ou pour la préparation d'une composition pharmaceutique, d'une quantité efficace de principe actif tel que décrit précédemment, c'est à dire un ou plusieurs peptides de séquence SEQ ID n°1 à SEQ ID n°8.

Selon un mode de réalisation particulièrement avantageux, la composition le contenant selon l'invention contient le peptide de séquence SEQ ID n°4.

Selon un autre mode de réalisation particulièrement avantageux, la composition selon l'invention contient le peptide de séquence SEQ ID n°5.

L'invention a également pour objet l'utilisation dans une composition cosmétique, ou pour la préparation d'une composition pharmaceutique, d'une quantité efficace de principe actif tel que décrit précédemment, le principe actif ou la composition le contenant étant destinés à induire, restaurer ou stimuler la pigmentation naturelle de la peau, des poils ou des cheveux.

L'invention se rapporte encore à l'utilisation dans une composition cosmétique, ou pour la préparation d'une composition pharmaceutique, d'une quantité efficace de principe actif tel que décrit précédemment, le principe actif ou la composition le contenant étant destinés, à préparer la peau à une exposition au soleil.

L'invention se rapporte encore à l'utilisation dans une composition cosmétique, d'une quantité efficace de principe actif tel que décrit précédemment, le principe actif ou la composition le contenant étant destinés, par l'augmentation de la synthèse de mélanine, à protéger la peau des rayonnements du soleil.

L'invention se rapporte encore à l'utilisation dans une composition cosmétique, d'une quantité efficace de principe actif tel que décrit précédemment, le principe actif ou la composition le contenant étant destinés à améliorer l'intensité et/ou l'homogénéité et/ou la persistance de la pigmentation de la peau et/ou des cheveux.

L'invention a, en outre, pour objet l'utilisation dans une composition cosmétique, d'une quantité efficace de principe actif tel que décrit précédemment, le principe actif ou la composition le contenant étant destinés à protéger la peau et les phanères des stress que produisent sur eux l'environnement. Plus précisément, la présente invention vise l'utilisation d'au moins un principe actif tel que défini précédemment, afin de protéger la peau et/ou les phanères contre tous les types d'agressions extérieures. On entend par le terme "agression extérieure" les agressions que peut produire l'environnement. Ces agressions peuvent être d'origine chimique, physique, biologique ou thermique. A titre d'exemple, on peut citer des agressions telles que la pollution, les UV, les frottements, l'eau à forte concentration de calcaire, les variations de température ou encore les produits à caractère irritant tels que les tensioactifs, les conservateurs ou les parfums.

Par phanères, on entend l'ensemble des annexes tégumentaires et notamment les ongles, les poils et les cheveux. Par poils et cheveux on entend l'ensemble des annexes pileuses et notamment également les cils et les sourcils.

Par ailleurs, les peptides selon l'invention, ou la composition les contenant, ont des effets anti-inflammatoires et anti-irritants. L'utilisation des propriétés de ce principe actif permet donc d'avoir une peau plus protégée et nettement moins sensible aux diverses agressions qu'elle peut rencontrer. La peau est ainsi apaisée.

Les peptides correspondant à la séquence SEQ ID n°1 à n°8, sont ainsi utilisés pour la fabrication d'une composition pharmaceutique à usage topique. Ils seront utilisés d'une manière plus générale afin de traiter les affections dermatologiques liées à la pigmentation. A ce titre, on peut citer par exemple les pathologies comme le vitiligo qui est une maladie auto-immune se caractérisant par l'apparition sur la peau de plaques blanches liées à un déficit de pigmentation ou encore le pityriasis versicolor, une mycose superficielle provoquant l'apparition de tâches claires pouvant se manifester d'emblée ou après une exposition au soleil, ou encore certaines pathologies associées au vieillissement chronologique ou actinique.

Ainsi, selon un autre aspect, les peptides selon l'invention, tels que décrits précédemment, pourront être utilisés pour la fabrication d'un médicament destiné au traitement des affections dermatologiques.

Selon un autre aspect, la présente invention concerne un procédé cosmétique pour augmenter la synthèse de mélanine dans les mélanocytes et favoriser la pigmentation naturelle de la peau et des cheveux, consistant à appliquer, sur la peau ou les cheveux une quantité efficace de principe actif, ou de la composition cosmétique le contenant, telle que définie précédemment, afin d'obtenir l'action désirée.

L'invention concerne encore un procédé de traitement cosmétique destiné à prévenir ou à traiter le blanchiment des cheveux et des poils, consistant à appliquer, sur la peau, la composition telle que définie précédemment.

L'invention concerne encore un procédé de traitement cosmétique pour les soins de la peau et/ou des phanères consistant à appliquer sur la surface de la peau une quantité efficace de principe actif, ou de la composition cosmétique le contenant, telle que définie précédemment, afin d'obtenir l'action désirée.

Le procédé de traitement cosmétique de l'invention peut être mis en oeuvre notamment en appliquant les compositions cosmétiques telles que définies ci-dessus, selon la technique d'utilisation habituelle de ces compositions, par exemple : application de crèmes, de gels, de sérums, de lotions, de laits, de shampooings ou de compositions anti-solaires, sur la peau ou sur les cheveux.

Des modes de réalisation particuliers de ce procédé de traitement cosmétique résultent également de la description précédente. D'autres avantages et caractéristiques de l'invention apparaîtront mieux à la lecture des exemples donnés à titre illustratif et non limitatif.

### Exemple 1 : Etude ex vivo de l'effet du peptide selon l'invention sur la synthèse de mélanine

Le but de cette étude *ex vivo* est de mettre en évidence l'augmentation de la mélanisation apportée par les peptides selon l'invention.

Protocole : Des biopsies de peau humaine de 6 mm de diamètre sont maintenues en culture *ex vivo* en présence d'un milieu spécifique (DMEM 1g/L, HAMF12, SVF et antibiotiques) sur des inserts déposés dans des plaques 6 puits. Les biopsies reçoivent ou non 2 applications par jour du peptide de séquence SEQ ID n°4 à une concentration à 1 % à partir d'une solution à 50 ppm. La durée de traitement est de 48 heures. Les biopsies sont ensuite fixées dans le formol 9 %, NaCl (150 mM) pendant 10 heures puis incluses dans la paraffine. Des coupes de peau de 3 µm d'épaisseur sont ensuite réalisées et la mélanine est colorée spécifiquement par la technique de Fontana-Masson.

Résultats : Les coupes de peau n'ayant pas reçu l'application de composé présentent une coloration de faible intensité. Au contraire, les coupes de peau ayant reçu les applications du peptide de séquence SEQ ID n° 4 présentent une coloration d'intensité nettement augmentée. De plus, la mélanine est située dans la couche basale, mais aussi transportée dans les couches supra-basales.

Conclusions : Le peptide de séquence SEQ ID n°4 induit une forte augmentation de la synthèse de mélanine par les mélanocytes et stimule l'ensemble du processus de répartition de mélanine dans l'épiderme.

### Exemple 2 : Préparation de compositions

**1 - Crème protection solaire:**

| ***Noms commerciaux*** | ***Noms INCI*** | ***% massique*** |
|---|---|---|
| ***PHASE A*** | | |
| Eau déminéralisée | Aqua (Water) | qsp |
| Pemulen TR1 | Acrylates/C10-30 Alkyl Acrylate Crosspolymer | 0,40 |
| Glycerine | Glycerin | 3,00 |
| Nipastat Sodium | Sodium Methylparaben (and) Sodium Ethylparaben (and) Sodium Butyl paraben (and) Sodium Propylparaben (and) Sodium Isobutylparaben | 0,15 |

| ***PHASE B*** | | |
|---|---|---|
| Parsol MCX | Ethylhexyl Methoxycinnamate | 7,50 |
| Eusolex 4360 | Benzophenone-3 | 3,00 |
| Parsol 1789 | Butyl Methoxydibenzoylmethane | 2,00 |
| Myritol 318 | Caprylic/Capric Triglyceride | 4,00 |
| Emulgade SEV | Hydrogenated Palm Glycerides (and) Ceteareth-20 (and) Ceteareth-12 (and) Cetearyl Alcohol | 5,00 |
| Propylparaben | Propylparaben | 0,15 |
| Nacol 16-98 | Cetyl Alcohol | 1,00 |

| ***PHASE C*** | | |
|---|---|---|
| TEA | Triethanolamine | 0,20 |

| ***PHASE D*** | | |
|---|---|---|
| Peptide séquence SEQ ID n°4 | | 0,1 ppm |

| ***Noms commerciaux*** | ***Noms INCI*** | ***% massique*** |
|---|---|---|
| Parfum | Parfum (Fragrance) | qsp |
| Colorant | | qsp |

Les constituants de la phase A et de la phase B sont chauffés séparément à une température comprise entre 70°C et 75°C. La phase B est émulsionnée dans A sous agitation. La phase C est ajoutée, à 45°C, en augmentant l'agitation. La phase D est ensuite additionnée lorsque la température se situe en dessous de 40°C. Le refroidissement est poursuivi jusqu'à 25°C sous vive agitation.

**2 -Crème auto-bronzante:**

| ***Noms commerciaux*** | ***Noms INCI*** | ***% massique*** |
|---|---|---|
| ***PHASE A*** | | |
| Eau déminéralisée | Aqua (Water) | qsp |
| Dipropylene Glycol | Dipropylene Glycol | 2,00 |
| Glycerine | Glycerin | 2,00 |
| Glydant Plus Liquid | DMDM Hydantoin (and) Iodopropynyl Butylcarbamate | 0,40 |

| ***PHASE B*** | | |
|---|---|---|
| Emulgade SEV | Glyceryl Stearate (and) Ceteareth-20/Ceteareth -12 (and) Cetearyl Alcohol (and) Cetyl Palmitate | 8,00 |
| Lanette O | Cetearyl Alcohol | 1,50 |
| Cetiol V | Decyl Oleate | 5,00 |
| DC 200 | Cyclomethicone | 2,00 |
| Cetiol SN | Cetearyl Isononanoate | 4,00 |
| Isopropyl Palmitate | Isopropyl Palmitate | 3,00 |
| Myritol 318 | Caprylic/Capric Triglyceride | 4,00 |
| Tocopheryl Acetate | Tocopheryl Acetate | 0,50 |

| ***PHASE C*** | | |
|---|---|---|
| Peptide séquence SEQ ID n°5 | | 5 ppm |

| ***PHASE D*** | | |
|---|---|---|
| Eau déminéralisée | Aqua (Water) | 10,00 |
| Dihydroxyacetone | Dihydroxyacetone | 5,00 |

| ***PHASE E*** | | |
|---|---|---|
| Colorant Caramel W 8016 | Caramel | 0,02 |
| Parfum | Parfum (Fragrance) | qsp |

Les phases A et B sont chauffées séparément sous agitation à 75°C. Émulsionner ensuite la phase B dans la phase A sous forte agitation. Introduire les peptides en dessous de 40°C. Puis, introduire la phase D préalablement solubilisée à froid. Colorer et parfumer.

Ajuster le pH si nécessaire à 4-4,5.

**3 -Spray auto-bronzant :**

| ***Noms commerciaux*** | ***Noms INCI*** | ***% massique*** |
|---|---|---|
| ***PHASE A*** | | |
| Eau déminéralisée | Aqua (Water) | qsp |
| Propylene Glycol | Propylene Glycol | 5,00 |
| Glycerine | Glycerin | 2,00 |
| Allantoine | Allantoin | 0,20 |

| ***PHASE B*** | | |
|---|---|---|
| Keltrol RD | Xanthan Gum | 0,05 |

| ***PHASE C*** | | |
|---|---|---|
| Glydant Plus Liquid | DMDM Hydantoin (and) Iodopropynyl Butylcarbamate | 0,40 |

| ***PHASE D*** | | |
|---|---|---|
| Peptide séquence SEQ ID n°4 | | 1 ppm |

| ***PHASE E*** | | |
|---|---|---|
| Dihydroxyacetone | Dihydroxyacetone | 5,00 |
| Eau déminéralisée | Aqua (Water) | 10,00 |

| ***PHASE F*** | | |
|---|---|---|
| Colorant Caramel E 105C | Caramel | qsp |
| Parfum | Parfum (Fragrance) | qsp |

Préparer la phase A sous agitation. Incorporer la gomme xanthane progressivement sous agitation défloculeuse. Les phases C et D seront incorporées une fois le gel terminé. La phase E préparée préalablement jusqu'à parfaite dissolution de la DHA sera rajoutée à la suite. Ajuster le pH si nécessaire à 4-4,5. Colorer et parfumer.

**4 -Lait après-soleil :**

| ***Noms commerciaux*** | ***Noms INCI*** | ***% massique*** |
|---|---|---|
| ***PHASE A*** | | |
| Montanov L | C14-22 Alcohols (and) C 12-20 Alkyl Glucoside | 3,00 |
| Waglinol 2559 | Cetearyl Isononanoate | 4,00 |
| Tegosoft TN | C12-15 Alkyl Benzoate | 3,00 |
| Huile de Noyaux d'Abricot | Prunus Armeniaca (Apricot) Kernel Oil | 2,00 |
| Huile d'Avocat | Persea Gratissima (Avocado) Oil | 1,00 |
| Abil 350 | Dimethicone | 1,00 |

| ***PHASE B*** | | |
|---|---|---|
| Eau déminéralisée | Aqua (Water) | qsp |

| ***PHASE C*** | | |
|---|---|---|
| Simulgel EG | Sodium Acrylate/Acryloyldimethyl Taurate Copolymer (and) Isohexadecane (and) Polysorbate 80 Copolymer (and) Polysorbate 80 | 0,4 |

| ***PHASE D*** | | |
|---|---|---|
| Phenonip | Phenoxyethanol (and) Methylparaben (and) Ethylparaben (and) Butylparaben (and) Propylparaben (and) Isobutylparaben Ethylparaben and Propylparaben and Buthylparaben | 0,30 |
| Germall 115 | Imidazolidinyl Urea | 0,20 |

| ***PHASE E*** | | |
|---|---|---|
| Peptide séquence SEQ ID n°4 | | 0,5 ppm |

Préparer la phase A sous agitation. Incorporer la gomme xanthane progressivement sous agitation défloculeuse. Les phases C et D seront incorporées une fois le gel terminé. La phase E préparée préalablement jusqu'à parfaite dissolution de la DHA sera rajoutée à la suite. Ajuster le pH si nécessaire à 4-4,5. Colorer et parfumer.

## Revendications

1. Composition cosmétique ou pharmaceutique, **caractérisée en ce qu'**elle contient, dans un milieu physiologiquement acceptable, un peptide de séquence :
(SEQ ID n°1) Asn-Gly-Trp-Lys-Ile-Glu-arg-Lys
(SEQ ID n°2) Asn-Gly-Trp-Arg-Val-Asp
(SEQ ID n°3) Trp-Arg-Leu-Asp-Arg-Lys
(SEQ ID n°4) Trp-Lys-Leu-Asp
(SEQ ID n°5) Trp-Arg-Val-Glu
(SEQ ID n°6) Trp-His-Leu-Glu
(SEQ ID n°7) Trp-Arg-Ala-Asp
(SEQ ID n°8) Trp-Lys-Ile-Asp.

2. Composition cosmétique ou pharmaceutique selon la revendication 1, **caractérisée en ce que** ledit peptide correspond à la séquence SEQ ID n° 4.

3. Composition cosmétique ou pharmaceutique selon la revendication 1, **caractérisée en ce que** ledit peptide correspond à la séquence SEQ ID n° 5.

4. Composition cosmétique ou pharmaceutique selon l'une quelconque des revendications précédentes, **caractérisée en ce que** ledit peptide possède au moins un groupement fonctionnel protégé par un groupement protecteur, ce groupement protecteur étant soit une acylation ou une acétylation de l'extrémité amino-terminale, soit une amidation ou une estérification de l'extrémité carboxy-terminale, soit les deux.

5. Composition selon l'une quelconque des revendications précédentes, **caractérisée en ce que** ledit peptide est préalablement solubilisé dans un ou plusieurs solvants cosmétiquement ou pharmaceutiquement acceptables, comme l'eau, le glycérol, l'éthanol, le propylène glycol, le butylène glycol, le dipropylène glycol, les diglycols éthoxylés ou propoxylés, les polyols cycliques, la vaseline, une huile végétale ou tout mélange de ces solvants.

6. Composition selon l'une quelconque des revendications précédentes, **caractérisée en ce que** ledit peptide est présent dans la composition à une concentration comprise entre 0,0005 et 500 ppm environ, et préférentiellement à une concentration comprise entre 0,01 et 5 ppm.

7. Composition selon l'une quelconque des revendications précédentes, **caractérisée en ce qu'**elle se présente sous une forme adaptée à l'application par voie topique comprenant un milieu cosmétiquement ou dermatologiquement acceptable.

8. Composition selon l'une quelconque des revendications précédentes, **caractérisée en ce que** ledit peptide est présent dans la composition en tant que principe actif, seul ou en association avec au moins un autre principe actif.

9. Composition selon l'une quelconque des revendications précédentes, **caractérisée en ce qu'**elle contient, en outre, au moins un colorant exogène des couches superficielles de l'épiderme et/ou au moins un principe actif pro-pigmentant diffèrent dudit peptide, choisi parmi les substrats de la tyrosinase ou les prostaglandines ou les composés activateurs de la voie de l'AMPc ou les extraits végétaux pigmentants.

10. Utilisation d'un peptide tel que défini dans l'une quelconque des revendications 1 à 4, dans une composition cosmétique ou pour la préparation d'une composition pharmaceutique.

11. Utilisation d'au moins un peptide tel que défini dans l'une quelconque des revendications 1 à 4, dans une composition cosmétique destinée à induire, restaurer ou stimuler la pigmentation naturelle de la peau, des poils ou des cheveux.

12. Utilisation d'au moins un peptide tel que défini dans l'une quelconque des revendications 1 à 4, dans une composition cosmétique destinée à préparer la peau à une exposition au soleil ou à protéger la peau des rayonnements du soleil.

13. Utilisation d'au moins un peptide tel que défini dans l'une quelconque des revendications 1 à 4, dans une composition cosmétique destinée à protéger la peau contre tous les types d'agressions extérieures.

14. Utilisation d'au moins un peptide tel que défini dans l'une quelconque des revendications 1 à 4, pour la préparation d'une composition pharmaceutique destinée à atténuer un désordre de la pigmentation de la peau, notamment le vitiligo.

15. Utilisation d'au moins un peptide tel que défini dans l'une quelconque des revendications 1 à 4, dans une composition cosmétique destinée à prévenir ou à traiter le blanchiment des poils et/ou des cheveux.

16. Procédé de traitement cosmétique **caractérisé en ce que** l'on applique topiquement sur la zone à traiter une composition telle que définie selon l'une quelconque des revendications 1 à 9.

## Patentansprüche

1. Kosmetische oder pharmazeutische Zusammensetzung, **dadurch gekennzeichnet, dass** sie in einem physiologisch akzeptablen Medium ein Peptid mit der folgenden Sequenz umfasst:
(SEQ ID Nr. 1) Asn-Gly-Trp-Lys-Ile-Glu-Arg-Lys
(SEQ ID Nr. 2) Asn-Gly-Trp-Arg-Val-Asp
(SEQ ID Nr. 3) Trp-Arg-Leu-Asp-Arg-Lys
(SEQ ID Nr. 4) Trp-Lys-Leu-Asp
(SEQ ID Nr. 5) Trp-Arg-Val-Glu
(SEQ ID Nr. 6) Trp-His-Leu-Glu
(SEQ ID Nr. 7) Trp-Arg-Ala-Asp
(SEQ ID Nr. 8) Trp-Lys-Ile-Asp.

2. Kosmetische oder pharmazeutische Zusammensetzung nach Anspruch 1, **dadurch gekennzeichnet, dass** das Peptid der Sequenz SEQ ID Nr. 4 entspricht.

3. Kosmetische oder pharmazeutische Zusammensetzung nach Anspruch 1, **dadurch gekennzeichnet, dass** das Peptid der Sequenz SEQ ID Nr. 5 entspricht.

4. Kosmetische oder pharmazeutische Zusammensetzung nach einem der vorhergehenden Ansprüche, **dadurch gekennzeichnet, dass** das Peptid mindestens eine funktionelle Gruppe aufweist, die von einer Schutzgruppe geschützt ist, wobei diese Schutzgruppe entweder eine Acylierung oder eine Acetylierung der amino-terminbalen Endung ist, oder eine Amidierung oder eine Veresterung des carboxy-terminalen Endes ist, oder beides.

5. Zusammensetzung nach einem der vorhergehenden Ansprüche, **dadurch gekennzeichnet, dass** das Peptid zuvor in einem oder in mehreren kosmetisch oder pharmazeutisch akzeptablen Lösemitteln löslich gemacht wird, wie z.B. Wasser, Glycerol, Ethanol, Propylenglycol, Butylenglycol, Dipropylenglycol, ethoxylierten oder propoxylierten Diglykolen, cyclischen Polyolen, Vaselin, einem pflanzlichen Öl oder jeder Mischung dieser Lösemittel.

6. Zusammensetzung nach einem der vorhergehenden Ansprüche, **dadurch gekennzeichnet, dass** das Peptid in der Zusammensetzung in einer Konzentration zwischen ungefähr 0,0005 und 500 ppm und vorzugsweise in einer Konzentration zwischen 0,01 und 5 ppm vorhanden ist.

7. Zusammensetzung nach einem der vorhergehenden Ansprüche, **dadurch gekennzeichnet, dass** sie in einer Form, die der Verabreichung auf topischem Weg angepasst ist, vorliegt, umfassend ein kosmetisch oder dermatologisch akzeptables Medium.

8. Zusammensetzung nach einem der vorhergehenden Ansprüche, **dadurch gekennzeichnet, dass** das Peptid in der Zusammensetzung als Wirkstoff, alleine oder in Verbindung mit mindestens einem anderen Wirkstoff, vorhanden ist.

9. Zusammensetzung nach einem der vorhergehenden Ansprüche, **dadurch gekennzeichnet, dass** sie außerdem mindestens einen exogenen Farbstoff der oberflächlichen Schichten der Epidermis umfasst, und/oder mindestens einen Pigmentierungswirkstoff der von dem Peptid verschieden ist, ausgewählt aus den Substraten der Tyrosinase oder der Prostaglandine, oder den Verbindungen zur Aktivierung des Wegs von cAMP oder den pflanzlichen Pigmentierungsextrakten.

10. Verwendung eines Peptids, wie in einem der Ansprüche 1 bis 4 definiert, in einer kosmetischen Zusammensetzung oder für die Zubereitung einer pharmazeutischen Zusammensetzung.

11. Verwendung mindestens eines Peptids, wie in einem der Ansprüche 1 bis 4 definiert, in einer kosmetischen Zusammensetzung, die ausgelegt ist um die natürliche Pigmentierung der Haut, der Körperhaare und des Haars zu induzieren, wiederherzustellen oder zu stimulieren.

12. Verwendung mindestens eines Peptids, wie in einem der Ansprüche 1 bis 4 definiert, in einer kosmetischen Zusammensetzung, die ausgelegt ist, um die Haut auf eine Aussetzung an die Sonne vorzubereiten oder die Haut vor den Strahlungen der Sonne zu schützen.

13. Verwendung mindestens eines Peptids, wie in einem der Ansprüche 1 bis 4 definiert, in einer kosmetischen Zusammensetzung, die ausgelegt ist, um die Haut vor allen Arten von äußeren Aggressionen zu schützen.

14. Verwendung mindestens eines Peptids, wie in einem der Ansprüche 1 bis 4 definiert, für die Zubereitung einer pharmazeutischen Zusammensetzung, die ausgelegt ist, um eine Störung der Pigmentierung der Haut, insbesondere das Vitiligo, zu lindern.

15. Verwendung mindestens eines Peptids, wie in einem der Ansprüche 1 bis 4 definiert, in einer kosmetischen Zusammensetzung, die ausgelegt ist, um der Bleichung der Körperhaare und/oder der Haare vorzubeugen oder sie zu behandeln.

16. Verfahren zur kosmetischen Behandlung, **dadurch gekennzeichnet, dass** topisch auf die zu behandelnde Zone eine Zusammensetzung aufgetragen wird, wie in einem der Ansprüche 1 bis 9 definiert.

## Claims

1. Cosmetic or pharmaceutical composition, **characterised in that** it contains, in a physiologically acceptable medium, a peptide having the sequence:
(SEQ ID No. 1) Asn-Gly-Trp-Lys-Ile-Glu-Arg-Lys
(SEQ ID No. 2) Asn-Gly-Trp-Arg-Val-Asp
(SEQ ID No. 3) Trp-Arg-Leu-Asp-Arg-Lys
(SEQ ID No. 4) Trp-Lys-Leu-Asp
(SEQ ID No. 5) Trp-Arg-Val-Glu
(SEQ ID No. 6) Trp-His-Leu-Glu
(SEQ ID No. 7) Trp-Arg-Ala-Asp
(SEQ ID No. 8) Trp-Lys-Ile-Asp.

2. Cosmetic or pharmaceutical composition according to claim 1, **characterised in that** said peptide corresponds to the sequence SEQ ID No. 4.

3. Cosmetic or pharmaceutical composition according to claim 1, **characterised in that** said peptide corresponds to the sequence SEQ ID No. 5.

4. Cosmetic or pharmaceutical composition according to any of the above claims, **characterised in that** said peptide has at least one functional group protected by a protecting group, this protecting group being either an acylation or an esterification of the carboxy-terminal end, or an amidation or an esterification of the carboxy-terminal end, or both.

5. Composition according to any of the above claims, **characterised in that** said peptide is previously solubilised in one or a plurality of cosmetically or pharmaceutically acceptable solvents, such as water, glycerol, ethanol, propylene glycol, butylene glycol, dipropylene glycol, ethoxylated or propoxylated diglycols, cyclic polyols, petroleum jelly, vegetable oil or any mixture of these solvents.

6. Composition according to any of the above claims, **characterised in that** said peptide is present in the composition at a concentration between approximately 0.0005 and 500 ppm, and preferentially at a concentration between 0.01 and 5 ppm.

7. Composition according to any of the above claims, **characterised in that** it is presented in a form suitable for topical application comprising a cosmetically or dermatologically acceptable medium.

8. Composition according to any of the above claims, **characterised in that** said peptide is present in the composition as an active agent, alone or in association with at least one further active agent.

9. Composition according to any of the above claims, **characterised in that** it further contains at least one exogenous dye of the superficial layers of the epidermis and/or at least one pro-pigmenting active agent different to said peptide, chosen from tyrosinase substrates or prostaglandins or cAMP pathway activating compounds or pigmenting plant extracts.

10. Use of a peptide as defined in any of claims 1 to 4, in a cosmetic composition or for preparing a pharmaceutical composition.

11. Use of at least one peptide as defined in any of claims 1 to 4, in a cosmetic composition for inducing, restoring or stimulating natural pigmentation of the skin or hair on the body or head.

12. Use of at least one peptide as defined in any of claims 1 to 4, in a cosmetic composition for preparing the skin for sun exposure or protecting the skin from sunrays.

13. Use of at least one peptide as defined in any of claims 1 to 4, in a cosmetic composition for protecting the skin against all types of external attacks.

14. Use of at least one peptide as defined in any of claims 1 to 4, in a pharmaceutical composition for attenuating a skin pigmentation disorder, notably vitiligo.

15. Use of at least one peptide as defined in any of claims 1 to 4, in a cosmetic composition for preventing or treating greying of hair on the body and/or head.

16. Cosmetic treatment method **characterised in that** a composition as defined according to any of claims 1 to 9 is applied topically onto the area to be treated.
